(19) Europäisches Patentamt
European Patent Office
Office européen des brevets

(11) Veröffentlichungsnummer: **0 189 035**
**A2**

(12) # EUROPÄISCHE PATENTANMELDUNG

(21) Anmeldenummer: **86100051.1**

(22) Anmeldetag: **03.01.86**

(51) Int. Cl.⁴: **C 07 D 227/04**, C 07 D 207/20, C 07 D 223/04

(30) Priorität: **23.01.85 DE 3502105**

(43) Veröffentlichungstag der Anmeldung: **30.07.86 Patentblatt 86/31**

(84) Benannte Vertragsstaaten: **DE FR GB IT**

(71) Anmelder: **BAYER AG, Konzernverwaltung RP Patentabteilung, D-5090 Leverkusen 1 Bayerwerk (DE)**

(72) Erfinder: **Schwarz, Hans-Helmut, Dr., Rather Strasse 90, D-4150 Krefeld (DE)**

(54) **Verfahren zur Herstellung von Schiff'schen Basen.**

㊇ Die Herstellung von aminogruppenhaltigen Schiff'schen Basen durch Umsetzung von cyclischen Lactamen oder deren ringgeöffneten Polymeren mit anorganischen Basen bei erhöhter Temperatur wird bewerkstelligt, indem man die Umsetzung in Gegenwart von inerten hochsiedenden Suspensionsmitteln durchführt.

EP 0 189 035 A2

BAYER AKTIENGESELLSCHAFT 5090 Leverkusen, Bayerwerk

Konzernverwaltung RP Bg/by-c
Patentabteilung

# Verfahren zur Herstellung von Schiff'schen Basen

Es ist bekannt, Schiff'sche Basen, die Aminogruppen enthalten, herzustellen durch Erhitzen von cyclischen Lactamen oder deren ringgeöffneten Polymeren, wie $\delta$-Valerolactam, $\varepsilon$-Caprolactam, n-Capryllactam, Nylon-4, Nylon-6 oder Nylon-8, bei Temperaturen unter 260°C in Gegenwart eines Erdalkalimetallhydroxids und nachfolgendes Erhitzen der Reaktionsmischung auf mindestens 300°C zur destillativen Abtrennung der Schiff'schen Basen.

Nach den in der DE-OS 1 420 044 und den Japanischen Anmeldungen 150 832, 42 088/66, 41 766/66 sowie in der DE-OS 230 334 beschriebenen Verfahren werden cyclische Lactame oder Polyamide, die aus den cyclischen Lactamen durch Polykondensation erhältlich sind, mit Basen, vorwiegend Calciumoxid, trocken auf Temperaturen im Bereich von 250 bis 500°C erhitzt und die flüchtigen Anteile abdestilliert.

Die Durchführung dieser Reaktion ist selbst im Laboratoriumsmaßstab mit erheblichen Schwierigkeiten verbunden, weil das Reaktionsgemisch im Laufe der Umsetzung in eine feste, harte Masse übergeht, aus der die flüchtigen Anteile abdestilliert werden müssen. Dabei fallen Rührkessel und ähnliche Reaktoren für die Durchführung dieser Reaktion aus. Nur aufwendige Apparate wie Kneter sind hierfür geeignet.

Eine Modifikation dieser Herstellungsmethode ist in dem Französischen Patent Nr. 1 475 526 beschrieben. Gemäß dieser Patentschrift wird die Reaktion zwischen den cyclischen Lactamen und den Erdalkalioxiden in einer Wirbelschicht durchgeführt. Dieses Verfahren stellt eine sehr aufwendige Technik dar, die durch hohe Investitionskosten und schwierige Fahrweise gekennzeichnet ist.

Es wurde nun ein einfaches Verfahren zur Herstellung von aminogruppenhaltigen Schiff'schen Basen der Formel

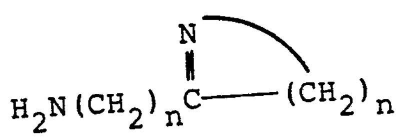

(I),

worin

n 3 bis 12 bedeutet,

durch Umsetzung von cyclischen Lactamen der Formel

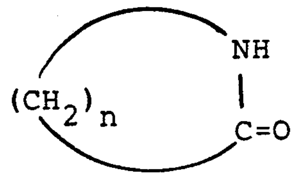

(II)

mit der obengenannten Bedeutung für n

oder deren ringgeöffneten Polymeren der Formel

$$-\left[-NH-(CH_2)_n-\underset{\overset{\|}{O}}{C}-\right]_m \qquad (III),$$

worin

n die obengenannte Bedeutung besitzt und

m für ganze Zahlen von 1 bis 100 000 steht,

mit anorganischen Basen bei erhöhter Temperatur gefunden, das dadurch gekennzeichnet ist, daß man die Umsetzung in Gegenwart von inerten hochsiedenden Suspensionsmitteln durchführt.

Als cyclische Lactame der Formel (II) werden in das erfindungsgemäße Verfahren bevorzugt solche mit n = 4 bis 12 eingesetzt. Beispielsweise seien als cyclische Lactame genannt: δ-Valerolactam, ε-Caprolactam, n-Capryllactam und Laurinlactam, bevorzugt ε-Caprolactam.

Als Polymere der Formel (III) werden bevorzugt solche in das erfindungsgemäße Verfahren eingesetzt in denen n für 4 bis 10 und m für 100 bis 400 steht. Genannt seien z.B. Polyamide, wie Nylon-4, Nylon-6 und Nylon-8, bevorzugt Nylon-6.

Die zur Suspension der Reaktionsteilnehmer geeigneten Stoffe müssen chemisch inert gegen die eingesetzten Reaktionspartner sein, bei den Reaktionstemperaturen sich in flüssigem Zustand befinden und einen so hohen Siedepunkt besitzen, daß nur geringe Anteile mit den Schiff'schen Basen und den Ausgangsprodukten abdestillieren.

Geeignete Suspensionsmittel sind beispielsweise hochsiedende, aliphatische und aromatische Kohlenwasserstoffe oder deren Gemische, hochsiedende polymere Kohlenwasserstoffe sowie hochsiedende Mineralöle, wie Heizöle und deren Destillationsrückstände.

Bevorzugt werden als inerte Suspensionsmittel hochsiedende Mineralöle in das erfindungsgemäße Verfahren eingesetzt.

Unter hochsiedenden Mineralölen werden hochsiedende Raffinerieprodukte verstanden mit einem Siedepunkt $\geq$ 250°C, wie Gasöl, Vakuumgasöl, Heizöl S, technisches Weißöl, geschmolzenes Paraffinwachs oder aromatisches Kohlenwasserstofföl. Vorteilhaft wird Vakuumgasöl mit einem Siedepunkt von über 300°C, insbesondere mit einem Siedebereich von 350°C bis 500°C verwendet.

Das erfindungsgemäße Verfahren wird im allgemeinen so ausgeführt, daß man in einem Rührkolben oder Rührkessel, der bei größeren Einheiten einen wandnahen Rührer besitzen soll, das cyclische Lactam oder dessen ringgeöffnetem Polymeren, die anorganische Base und das Suspensionsmittel einfüllt. Durch Erwärmen der Reaktionsmischung auf etwa 270 bis 400°C, bevorzugt 300 bis 360°C, wird die gebildete Schiff'sche Base gegebenenfalls im Vakuum abdestilliert.

Als anorganische Base wird besonders bevorzugt das Calciumoxid in das erfindungsgemäße Verfahren eingesetzt. Es ist jedoch auch möglich andere Erdalkalioxide und/oder -hydroxyde sowie Alkalioxide und/oder -hydroxide, wie Natriumhydroxid, Kaliumhydroxid, Strontium- und/oder Bariumoxid sowie Lithiumcarbonat in das erfindungsgemäße Verfahren einzusetzen.

Das Mengenverhältnis von cyclischem Lactam oder dessen ringgeöffnetem Polymeren einerseits zu den einzusetzenden anorganischen Basen andererseits kann in weiten Grenzen variiert werden. Es empfiehlt sich jedoch, mindestens 0,5 Mol einer anorganischen Base (z.B. CaO) auf 1 Mol des eingesetzten Lactams oder dessen ringgeöffnetem Polymeren einzusetzen, da bei geringeren Mengen an anorganischer Base ungenügende Ausbeuten an Schiff'schen Basen erhalten werden. Bevorzugt werden 0,55 bis 0,95 Mol

einer anorganischen Base (z.B. CaO bezogen auf 1 Mol des eingesetzten cyclischen Lactams oder dessen ringgeöffnetem Polymeren in das erfindungsgemäße Verfahren eingesetzt.

Die Menge an inerten hochsiedendem Suspensionsmittel sollte, bezogen auf das Gemisch aller Einsatzkomponenten, nicht unter 25 Gew.-% liegen, weil dann unter Umständen die Mischung nicht bei allen Reaktionszuständen rührbar ist. Bevorzugt werden 30 bis 70 Gew.-% an hochsiedendem Suspensionsmittel, bezogen auf das Gemisch aller Einsatzkomponenten, in das erfindungsgemäße Verfahren eingesetzt. Sollten während der Reaktion Teile des Suspensionsmittels abdestillieren, so kann zur Aufrechterhaltung eines rührfähigen Zustandes auch während der Umsetzung Suspensionsmittel nachdosiert werden.

Es kann zweckmäßig für das erfindungsgemäße Verfahren sein, den Aufheizvorgang im geeigneten Temperaturbereich zu verlangsamen, um den Lactamen eine Polykondensation zu ermöglichen. Bei Verwendung von beispielsweise ε-Caprolactam beträgt dieser Temperaturbereich etwa 220 bis 250°C.

Der Einsatz von inerten hochsiedenden Suspensionsmitteln bei dem erfindungsgemäßen Verfahren ermöglicht es aminogruppenhaltige Schiff'sche Basen in technisch einfacher und wirtschaftlicher Weise herzustellen. Durch den Einsatz von Suspensionsmitteln erhält man ein rührfähiges Reaktionsgemisch, das in allen Stadien der Reaktion in normalen Rührkesseln verarbeitet werden kann, ohne daß störende Zusammenbackungen der festen Reaktionsteilnehmer auftreten.

Eine Wiederverwendung der Suspensionsmittel ist nach Entfernung der anorganischen Bestandteile, z.B. durch Filtration oder Zentrifugation, möglich. Ein großer Reinigungseffekt wird erzielt, wenn man das Suspensionsmittel - vornehmlich Mineralöle - mit wäßrigen Mineralsäuren behandelt, die die Carbonate und Basen sowie organische basische Stoffe auflösen und die dabei entstehende wäßrige Phase abtrennt. Reste von Säuren können dann durch eine Wasser- oder Alkaliwäsche aus dem Suspensionsmittel entfernt werden. Ausgefallene Feststoffe können durch Filtration beseitigt werden.

Da Heizöle oder Vakuumgasöl wohlfeil zur Verfügung stehen, wird man diese Suspensionsmittel in der Regel einer Verfeuerung zuführen und die Reaktion mit frischem Mineralöl beschicken.

Die nachfolgenden Beispiele sollen das erfindungsgemäße Verfahren erläutern, ohne es jedoch auf diese Beispiele einzuschränken.

Beispiel 1

1412 g Caprolactam, 403,5 g CaO und 605 g Paraffinöl wurden im Rührkolben im Laufe von 3,2 h auf 315°C erhitzt. Zu diesem Zeitpunkt wurde der Kolbeninhalt auf einen Druck von 708 mbar eingestellt. Dabei begann das gebildete 7-(5-Aminopentyl)-3,4,5,6-tetrahydro-2H-azepin zu destillieren. Während der folgenden zwei Stunden wurde das Vakuum allmählich auf 175 mbar erniedrigt. Nachdem eine Destillatmenge von 700 ml angefallen war, wurden 878 g Paraffinöl in den Rührkolben eingepumpt.

Während dieses Prozesses fiel ein zweiphasiges Destillat an. Es bestand aus einer oberen paraffinischen Phase von 43 g. Die untere Phase (1010 g) enthielt nach Analyse 71,9 % Azepin und 10,3 % Caprolactam. Das entsprach einem Lactam-Umsatz von 92,6 %, einer Azepin-Selektivität von 68,9 % und einer Ausbeute von 63,8 %, bezogen auf Caprolactam.

Beispiel 2

182,8 g 2-Pyrrolidon, 69,4 g CaO, 260 g Paraffinöl wurden im Rührkolben 1 h lang auf 220° erhitzt. Man erhöhte anschließend die Temperatur auf 310 bis 320° und destillierte bei 25 mbar. Das Destillat bestand aus 2 Phasen. Die obere (68,6 g) bestand aus Paraffinöl. Die untere (91,43 g) enthielt nach GC-Analyse 36,9 g nicht umgesetztes Pyrrolidon und 44,3 g 3,4-Dihydro-2H-pyrrol-5-propanamin. Das entsprach einem Pyrrolidon-Umsatz von 79,7 % und einer Ausbeute von 40,9 %.

Beispiel 3

Nach der Arbeitsweise des Beispiels 1 wurden 429,4 g Polycaprolactamgranulat (m = 100-400) (Durethan BK ® der Bayer AG), 112 g CaO und 260 g technisches Weißöl (Catenexöl P 941 ® der Shell AG) umgesetzt. Es entstand ein zweiphasiges Destillat, das aus 196,5 g einer Paraffin- und 341,3 g einer Azepinphase bestand. Durch Analyse wurden in dem Gemisch 245,5 g 7-(5-Aminopentyl)-3,4,5,6-tetrahydro-2H-azepin und 73,5 g Lactam festgestellt. Das entsprach einem Polyamidumsatz von 82,9 % und einer Azepin-Selektivität von 85,6 % und einer Azepin-Ausbeute bezogen auf Polyamid von 70,1 %.

Beispiel 4

Entsprechend der Arbeitsweise des Beispieles 2 wurden 243 g Caprolactam, 104,1 g CaO und 260 g Heizöl S umgesetzt. Es wurden 280,3 g eines Destillats gewonnen, das nach GC-Analyse 128,1 g 7-(5-Aminopentyl)-3,4,5,6-tetrahydro-2H-azepin und 36 g Caprolactam enthielt. Das entsprach einem Lactam Umsatz von 85,2 % und einer Azepin-Ausbeute von 77,2 %.

Patentansprüche

1. Verfahren zur Herstellung von aminogruppenhaltigen Schiff'schen Basen der Formel

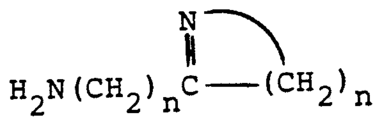,

worin n 3 bis 12 bedeutet,

durch Umsetzung von cyclischen Lactamen der Formel

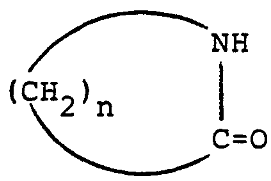

mit der obengenannten Bedeutung für n

oder deren ringgeöffneten Polymeren der Formel

$$-[\overline{N}H-(CH_2)_n-\underset{\underset{O}{\|}}{C}-]_m ,$$

worin

n die obengenannte Bedeutung besitzt und
m für ganze Zahlen von 1 bis 100 000 steht,

mit anorganischen Basen bei erhöhter Temperatur, dadurch gekennzeichnet, daß man die Umsetzung in Gegenwart von inerten hochsiedenden Suspensionsmitteln durchführt.

2. Verfahren nach Anspruch 1, dadurch gekennzeichnet, daß man als inertes Suspensionsmittel hochsiedende Mineralöle einsetzt.

3. Verfahren nach Ansprüchen 1 und 2, dadurch gekennzeichnet, daß man als hochsiedende Mineralöle Gasöl, Vakuumgasöl, Heizöl S, technisches Weißöl, geschmolzenes Paraffinwachs oder aromatisches Kohlenwasserstofföl mit einem Siedepunkt von $\geq$ 250°C einsetzt.

4. Verfahren nach Ansprüchen 1 bis 3, dadurch gekennzeichnet, daß man Vakuumgasöl einsetzt, das einen Siedebereich von 350 bis 500°C besitzt.